# EUROPEAN PATENT APPLICATION

(11) **EP 3 392 332 A1**
(43) Date of publication of application: **24.10.2018**
(21) Application number: 16875673.2
(22) Date of filing: 14.12.2016
(51) Int. Cl.: C12M 1/00, C12M 3/00

(54) **CELL CULTURE DEVICE AND CELL CULTURE SYSTEM**

(30) Priority: 15.12.2015 JP 2015243745; 23.09.2016 JP 2016185835
(71) Applicant: OLYMPUS CORPORATION, Hachioji-shi Tokyo 192-8507 (JP)
(72) Inventor: SASAKI, Hiroshi, Hachioji-shi Tokyo 192-8507 (JP); MINAMI, Tatsuya, Hachioji-shi Tokyo 192-8507 (JP); MAKARA, Yasunori, Hachioji-shi Tokyo 192-8507 (JP)
(74) Representative: Winter, Brandl, Fürniss, Hübner, Röss, Kaiser, Polte - Partnerschaft mbB
(86) International application number: PCT/JP2016/087194
(87) International publication number: WO 2017/104696

(57) **Abstract**

Provided is a cell-culturing apparatus (100) including: an incubator (5) that can maintain an interior thereof in an envir onment that is appropriate for growth of cells; a cell-culturing vessel (4) that is accommodated inside the incubator (5); an op tical-data acquisition unit (1) that acquires optical data of a medium in the cell-culturing vessel (4); a medium changing unit (2) that changes the medium in the cell-culturing vessel (4); an d a controlling means (3), wherein a timing at which the medium is changed is determined on the basis of a change over time in t he optical data acquired by the optical-data acquisition unit (1 )

## Description

### {Technical Field}

The present invention relates to a cell-culturing apparatus and a cell-culturing system with which it is possible to automatically change a medium in a cell-culturing vessel.

### {Background Art}

In recent years, in accordance with advances being made in stem-cell research and regenerative medicine, there has been a demand for preparing a large amount of cells. During culturing, cells take up components required for growth, such as oxygen, nutrients, and so forth, and discharge carbon dioxide and waste products. Therefore, it is necessary to periodically change a medium because the medium deteriorates when cells are cultured for a long period of time, and thus, an operator must constantly check the degree of deterioration of the medium. However, because it is necessary to move a sample into/out of an incubator in order for the operator to check the degree of deterioration of the medium, when doing so, cells experience stresses, for example, changes in environment such as temperature or the like and impacts caused when being conveyed, and thus, the growth of the cells may be affected.

As a system that automatically changes a medium, there is a known system in which a culturing vessel is moved, by means of a conveying robot, between an incubator and a medium-changing robot (for example, see Patent Literature 1).

### {Citation List}

### {Patent Literature}

{PTL 1} Japanese Unexamined Patent Application, Publication No. 2002-262856

### {Summary of Invention}

### {Technical Problem}

With an automatic medium-changing system, such as that in Patent Literature 1, the system becomes extremely complicated because a conveying robot or the like is employed, and thus, it is highly likely that system errors occur. In addition, because a cell-culturing vessel is moved into/out of an incubator, cells in the cell-culturing vessel experience stresses, for example, changes in environment such as temperature or the like and impacts caused when being conveyed. Because, if the system is installed in the incubator in order to avoid such stresses, problems may occur in mechanical and electrical structures due to the high-humidity environment in the incubator, it is preferable that system components in the incubator be configured as simply as possible.

The present invention has been conceived in light of the above-described circumstances, and an object thereof is to provide a cell-culturing apparatus and a cell-culturing system with which it is possible to change a medium in a cell-culturing vessel in a simple, timely manner in accordance with the degree of deterioration of the medium, and to reduce the occurrence of system problems by employing a simple configuration.

### {Solution to Problem}

In order to achieve the above-described object, the present invention provides the following solutions.

An aspect of the present invention is a cell-culturing apparatus including: an incubator that can maintain an interior thereof in an environment that is appropriate for growth of cells; a cell-culturing vessel that is accommodated inside the incubator and that holds cells and a medium therein; an optical-data acquisition unit that acquires optical data of the medium in the cell-culturing vessel; a medium changing unit that changes the medium in the cell-culturing vessel; and a controlling means, wherein the optical-data acquisition unit is provided with an irradiation optical system that radiates monochromatic light onto the medium in the cell-culturing vessel and a measurement optical system that measures a light intensity of the monochromatic light that has passed through the medium in the cell-culturing vessel wherein the medium changing unit is provided with a medium-supplying means for supplying the medium to the cell-culturing vessel and a medium-discharging means for discharging the medium from the cell-culturing vessel, and wherein the controlling means detects a change over time in the light intensity measured by the measurement optical system, and controls the medium-supplying means and/or the medium-discharging means on the basis of said change over time.

With this aspect, it is possible to change the medium in the cell-culturing vessel in a timely manner in accordance with the deterioration of the medium by employing a simple configuration.

Another aspect of the present invention is a cell-culturing apparatus including: an incubator that can maintain an interior thereof in an environment that is appropriate for growth of cells; a cell-culturing vessel that is accommodated inside the incubator and that holds cells and a medium therein; an optical-data acquisition unit that acquires optical data of the medium in the cell-culturing vessel; a medium changing unit that changes the medium in the cell-culturing vessel; and a controlling means, wherein the optical-data acquisition unit is provided with an irradiation optical system that radiates monochromatic light onto the medium in the cell-culturing vessel, a beam splitter that splits the monochromatic light radiated from the irradiation optical system into two beams, a first measurement optical system that, of the monochromatic light beams split by the beam splitter, measures a light intensity of one of the monochromatic light beams before passing through the medium in the cell-culturing vessel, and a second measurement optical system that, of the monochromatic light beams split by the beam splitter, measures a light intensity of the other monochromatic light beam that has passed through the medium in the cell-culturing vessel, wherein the medium changing unit is provided with a medium-supplying means for supplying the medium to the cell-culturing vessel and a medium-discharging means for discharging the medium from the cell-culturing vessel, and wherein the controlling means calculates an amount of light absorbed by the medium on the basis of the light intensities measured by the first measurement optical system and the second measurement optical system, detects a change over time in the calculated light-absorption amount, and controls the medium-supplying means and/or the medium-discharging means on the basis of said change over time.

With this aspect, it is possible to change the medium in the cell-culturing vessel in a timely manner in accordance with the deterioration of the medium by employing a simple configuration.

Another aspect of the present invention is a cell-culturing apparatus including: an incubator that can maintain an interior thereof in an environment that is appropriate for growth of cells; a cell-culturing vessel that is accommodated inside the incubator and that holds cells and a medium therein; an optical-data acquisition unit that acquires optical data of the medium in the cell-culturing vessel; a driving means for moving the optical-data acquisition unit and the cell-culturing vessel relative to each other; a medium changing unit that changes the medium in the cell-culturing vessel; and a controlling means, wherein the optical-data acquisition unit is provided with an irradiation optical system that radiates monochromatic light onto the medium in the cell-culturing vessel and a measurement optical system that measures a light intensity of the monochromatic light radiated from the irradiation optical system, wherein the medium changing unit is provided with a medium-supplying means for supplying the medium to the cell-culturing vessel and a medium-discharging means for discharging the medium from the cell-culturing vessel, and wherein the controlling means causes the optical-data acquisition unit and the cell-culturing vessel to be moved relative to each other by means of the driving means, calculates an amount of light absorbed by the medium on the basis of a first light intensity measured when the cell-culturing vessel is not disposed in an optical path between the irradiation optical system and the measurement optical system and a second light intensity measured when the cell-culturing vessel is disposed in the optical path between the irradiation optical system and the measurement optical system, detects a change over time in the calculated light-absorption amount, and controls the medium-supplying means and/or the medium-discharging means on the basis of said change over time.

With this aspect, it is possible to change the medium in the cell-culturing vessel in a timely manner in accordance with the deterioration of the medium by employing a simple configuration.

In the above-described aspect, at least the cell-culturing vessel, the optical-data acquisition unit, and the controlling means may be disposed inside the incubator, and the controlling means may be provided with a transmitting/receiving portion that transmits/receives signals to/from outside the incubator, and, by transmitting/receiving, by means of the transmitting/receiving portion, the signals to/from an external controlling means installed outside the incubator, the optical-data acquisition unit and the medium changing unit may be remotely controlled.

By doing so, because it is possible to control this cell-culturing apparatus from outside the incubator, an operator can execute the medium change at an arbitrary timing. In addition, because it is not necessary to move the cell-culturing vessel into/out of the incubator, it is possible to reduce the influences on the cells.

In the above-described aspect, the irradiation optical system and the measurement optical system may be disposed in a top-to-bottom direction with the cell-culturing vessel interposed therebetween.

In the above-described aspect, the irradiation optical system and the measurement optical system may be disposed in a horizontal direction with the cell-culturing vessel interposed therebetween.

By doing so, it is possible to acquire the optical data in a state in which the cells are absent in the optical path, and thus, it is possible to eliminate the influences of the light absorption by the cells.

In the above-described aspect, the irradiation optical system and the measurement optical system may be disposed on the same side with respect to the cell-culturing vessel, a reflecting member may be provided on an opposite side of the irradiation optical system and the measurement optical system with the cell-culturing vessel interposed therebetween, and the measurement optical system may measure a light intensity of the monochromatic light that is radiated onto the cell-culturing vessel from the irradiation optical system, that is reflected by the reflecting member after passing through the cell-culturing vessel, and that has passed through the cell-culturing vessel again.

By doing so, it is possible to make the apparatus configuration compact, and it is made easier to dispose the apparatus in the incubator. In addition, because the monochromatic light is made to pass through the medium in the cell-culturing vessel twice, the amount of light absorbed by the medium is increased, and thus, the detection sensitivity for changes in the light-absorption amount is increased.

In the above-described aspect, the irradiation optical system may be provided with a white light source, and a band-pass filter that is provided so as to be insertable into/removable from an optical path of white light emitted from the white light source and that allows only light having a desired wavelength to pass therethrough.

By doing so, it is possible to radiate light having a desired wavelength just by changing the band-pass filter, and thus, the versatility of the apparatus is enhanced and the operability for the operator is also enhanced.

In the above-described aspect, the irradiation optical system may be provided with a plurality of monochromatic light sources, and a mirror and a dichroic mirror that combine optical paths of light coming from the plurality of monochromatic light sources.

By doing so, it is possible to measure the light-absorption amounts at multiple wavelengths that are different from each other, and thus, it is possible to more accurately detect the deterioration of the medium.

In the above-described aspect, the medium-supplying means may be provided with a medium holding means for holding the medium, a tubular member that connects the medium holding means and the cell-culturing vessel, and a liquid feeding pump disposed in the tubular member, and the liquid feeding pump may be controlled by means of the signals from the controlling means.

In the above-described aspect, the medium-discharging means may be provided with a waste-liquid holding vessel that holds the medium discharged from the cell-culturing vessel, a tubular member that connects the waste-liquid holding vessel and the cell-culturing vessel, and a liquid feeding pump disposed in the tubular member, and the liquid feeding pump may be controlled by means of the signals from the controlling means.

In the above-described aspect, the medium-discharging means may be provided with a waste-liquid holding vessel that holds the medium discharged from the cell-culturing vessel, a tubular member that connects the waste-liquid holding vessel and the cell-culturing vessel, and a negative-pressure supplying means for applying a negative pressure to the waste-liquid holding vessel, and the negative-pressure supplying means may be controlled by means of the signals from the controlling means.

By doing so, it is possible to change the medium by employing a simple configuration.

Another aspect of the present invention is a cell-culturing system including: a first cell-culturing apparatus that is selected from the cell-culturing apparatuses according to any one the above-described aspects, and a second cell-culturing apparatus that is provided with a second cell-culturing vessel that is accommodated inside the incubator of the first cell-culturing apparatus and that holds cells and a medium, and a second medium changing unit that changes the medium in the second cell-culturing vessel, wherein the controlling means of the first cell-culturing apparatus controls the medium changing unit of the first cell-culturing apparatus and the second medium changing unit of the second cell-culturing apparatus on the basis of the change over time.

With this aspect, even in the case in which cells are cultured by using a plurality of cell-culturing vessels, because it suffices to provide a single optical-data acquisition unit, it is possible to make the system compact and it is also advantageous in terms of costs.

### {Advantageous Effects of Invention}

The present invention affords an advantage in that it is possible to detect deterioration of a medium in a cell-culturing vessel disposed in an incubator and to change the medium in a timely manner in accordance with the deterioration of the medium. In addition, because the apparatus configuration is simple, it is possible to reduce the risk of a system error occurring, and thus, it is possible to avoid the possibility of an error affecting culturing conditions. In addition, it is possible to reduce the time and effort involved in work performed by an operator, and it is also possible to reduce the possibility of a cell-culturing system becoming contaminated in association with this work. Furthermore, because it is possible to change the medium without having to take out the cell-culturing vessel from the incubator, it is possible to reduce stresses experienced by the cells in association with changes in environment such as temperature or the like, and it is also possible to avoid impacts that act on the cells when conveying the cell-culturing vessel.

### {Brief Description of Drawings}

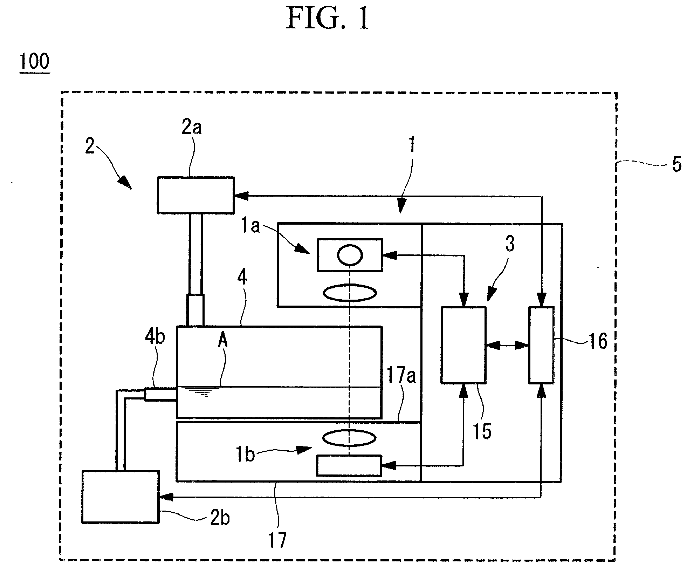

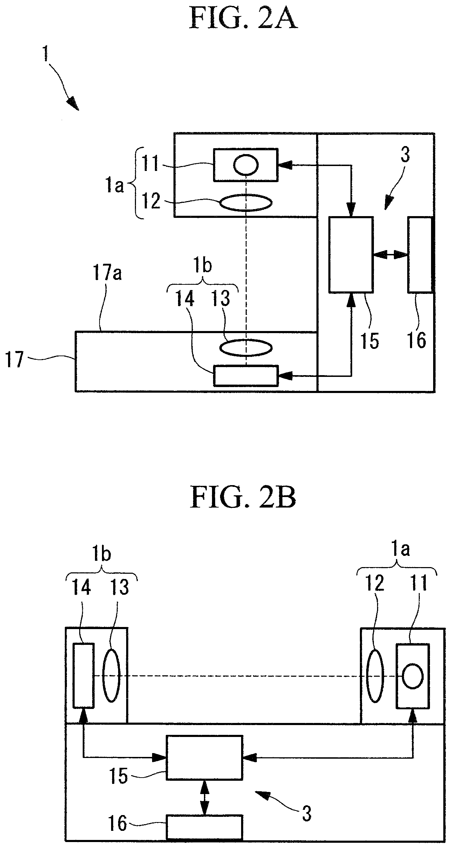

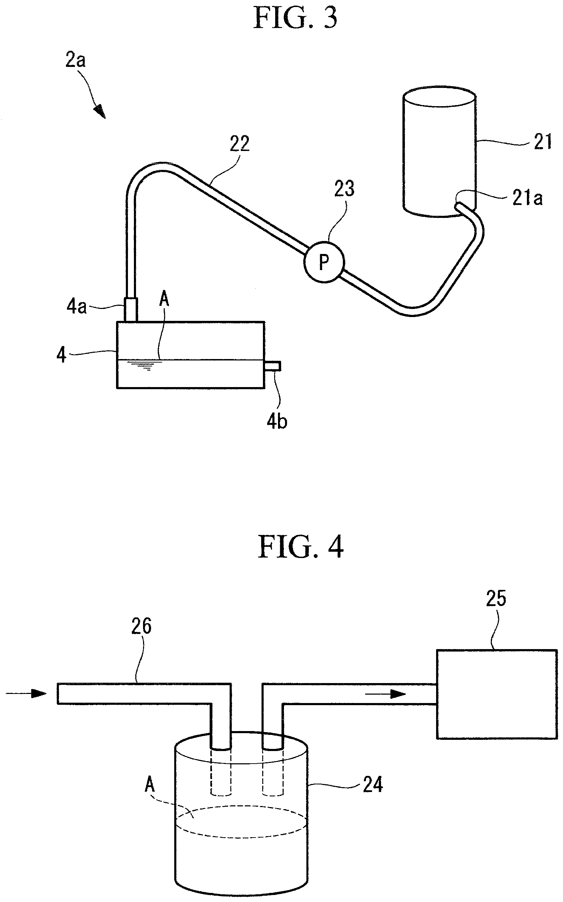

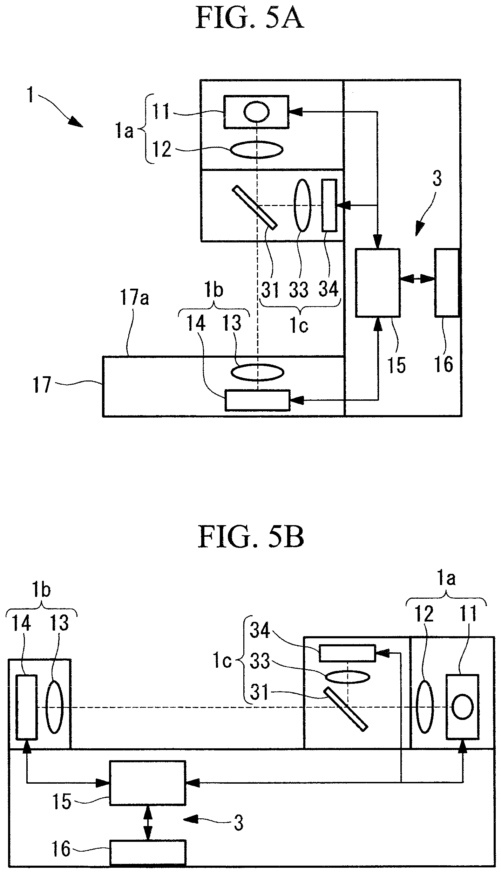

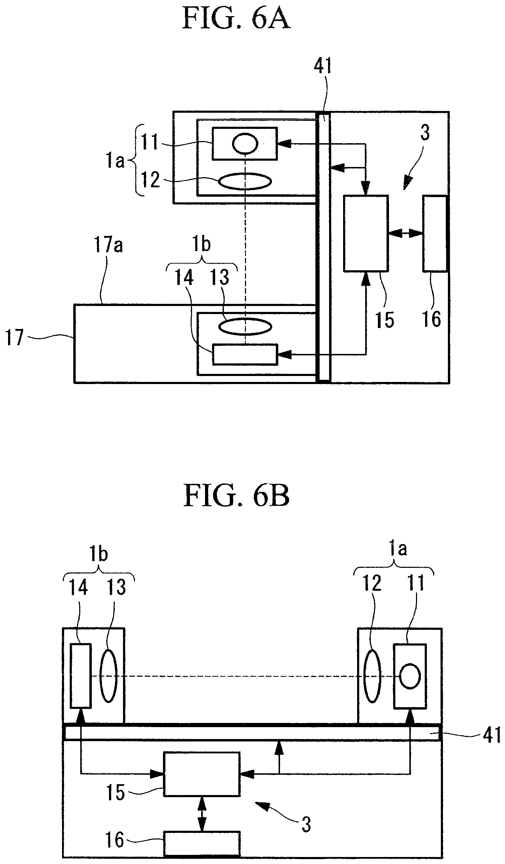

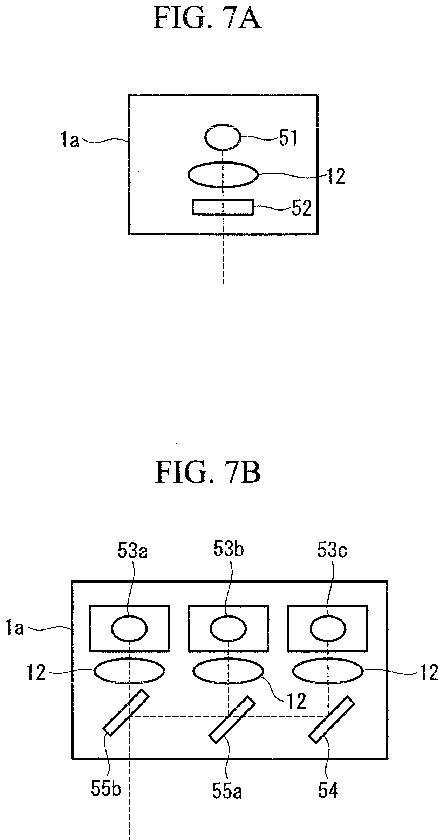

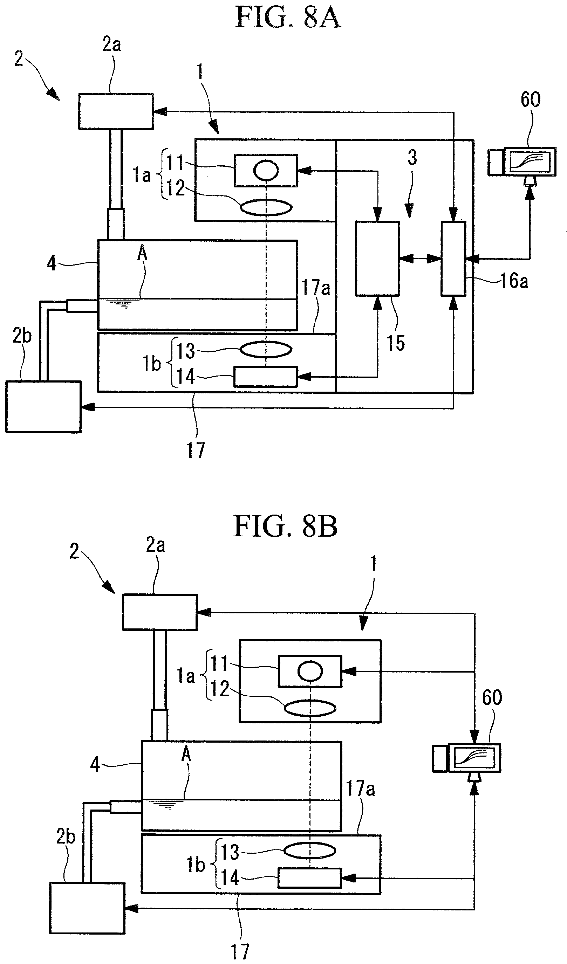

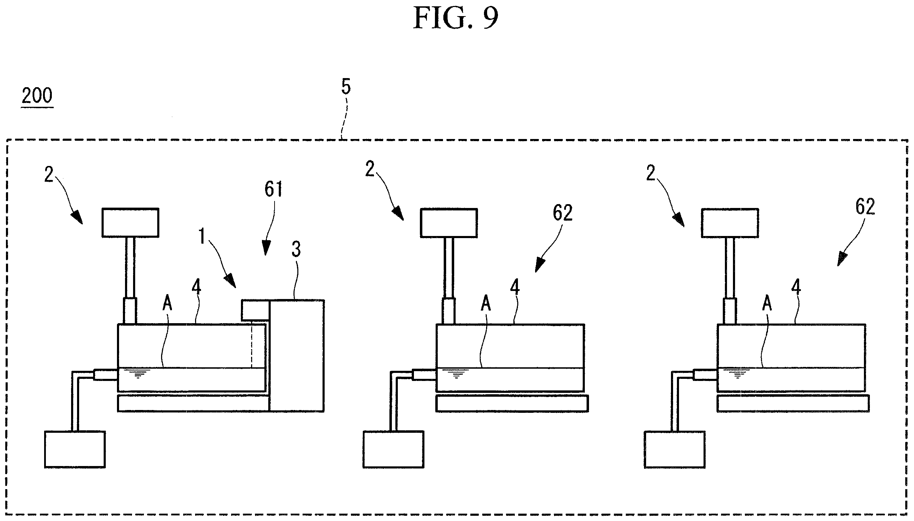

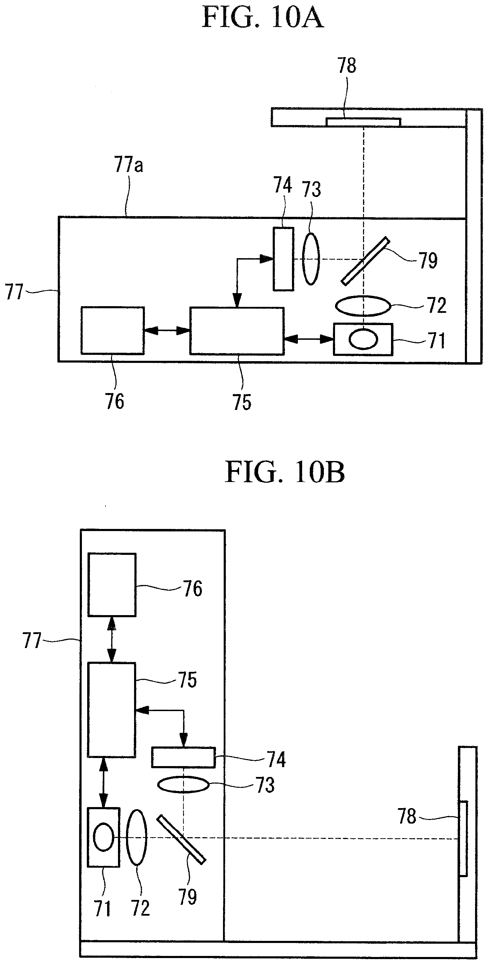

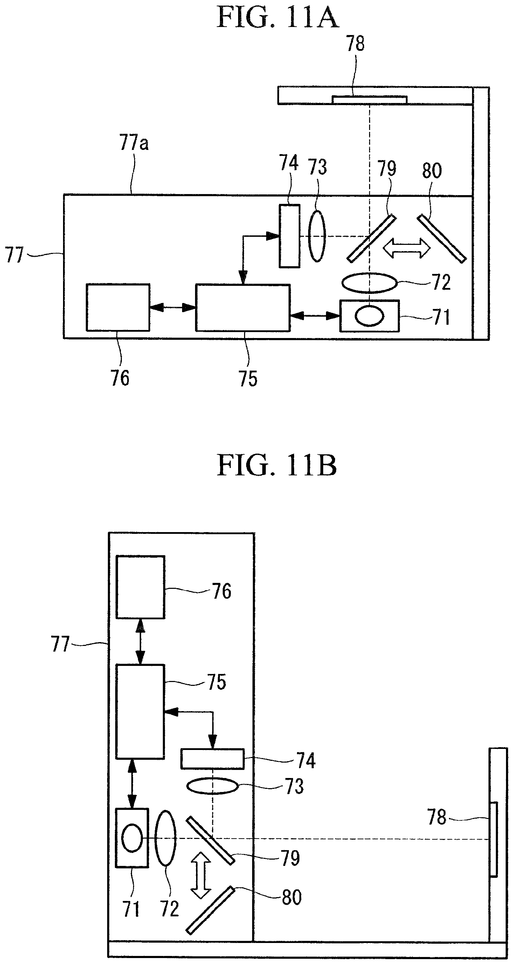

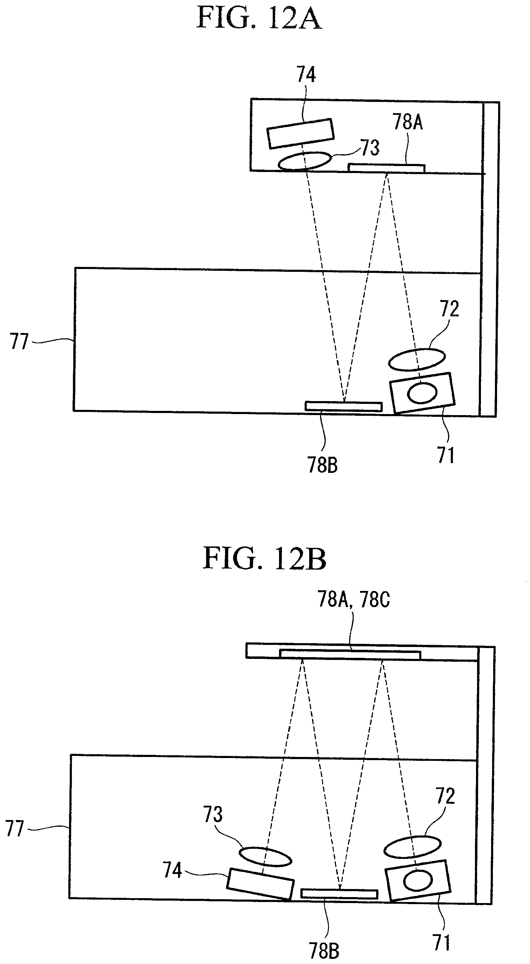

{Fig. 1} Fig. 1 is an explanatory diagram showing, in outline, the configuration of a cell-culturing apparatus according to a first embodiment of the present invention.
{Fig. 2A} Fig. 2A is an explanatory diagram showing, in outline, the configurations of an optical-data acquisition unit and a controlling means of the cell-culturing apparatus according to the first embodiment of the present invention.
{Fig. 2B} Fig. 2B is an explanatory diagram showing, in outline, the configurations of an optical-data acquisition unit and a controlling means of a cell-culturing apparatus according to a modification of the first embodiment of the present invention.
{Fig. 3} Fig. 3 is an explanatory diagram showing, in outline, the configuration of an example of a medium-supplying means of the present invention.
{Fig. 4} Fig. 4 is an explanatory diagram showing, in outline, the configuration of an example of a medium-discharging means of the present invention.
{Fig. 5A} Fig. 5A is an explanatory diagram showing, in outline, the configurations of an optical-data acquisition unit and a controlling means of a cell-culturing apparatus according to a second embodiment of the present invention.
{Fig. 5B} Fig. 5B is an explanatory diagram showing, in outline, the configurations of an optical-data acquisition unit and a controlling means of a cell-culturing apparatus according to a modification of the second embodiment of the present invention.
{Fig. 6A} Fig. 6A is an explanatory diagram showing, in outline, the configurations of an optical-data acquisition unit and a controlling means of a cell-culturing apparatus according to a third embodiment of the present invention.
{Fig. 6B} Fig. 6B is an explanatory diagram showing, in outline, the configurations of an optical-data acquisition unit and a controlling means of a cell-culturing apparatus according to a modification of the third embodiment of the present invention.
{Fig. 7A} Fig. 7A is an explanatory diagram showing, in outline, the configuration of a modification of an irradiation optical system of the present invention.
{Fig. 7B} Fig. 7B is an explanatory diagram showing, in outline, the configuration of a modification of the irradiation optical system of the present invention.
{Fig. 8A} Fig. 8A is an explanatory diagram showing, in outline, the configuration of a modification of a cell-culturing system of the present invention.
{Fig. 8B} Fig. 8B is an explanatory diagram showing, in outline, the configuration of a modification of the cell-culturing system of the present invention.
{Fig. 9} Fig. 9 is an explanatory diagram showing, in outline, the configuration of a modification of a cell-culturing system according to an embodiment of the present invention.
{Fig. 10A} Fig. 10A is an explanatory diagram showing, in outline, the configuration of a modification of the cell-culturing apparatus of the present invention.
{Fig. 10B} Fig. 10B is an explanatory diagram showing, in outline, the configuration of a modification of the cell-culturing apparatus of the present invention.
{Fig. 11A} Fig. 11A is an explanatory diagram showing, in outline, the configuration of a modification of the cell-culturing apparatus of the present invention.
{Fig. 11B} Fig. 11B is an explanatory diagram showing, in outline, the configuration of a modification of the cell-culturing apparatus of the present invention.
{Fig. 12A} Fig. 12A is an explanatory diagram showing, in outline, the configuration of a modification of the cell-culturing apparatus of the present invention.
{Fig. 12B} Fig. 12B is an explanatory diagram showing, in outline, the configuration of a modification of the cell-culturing apparatus of the present invention.

### {Description of Embodiments}

Cell-culturing apparatuses according to embodiments of the present invention will be described below with reference to the drawings.

### (First Embodiment)

A cell-culturing apparatus 100 according to a first embodiment of the present invention is an apparatus having a configuration shown in Fig. 1. The cell-culturing apparatus 100 is an apparatus that changes a medium A in a cell-culturing vessel 4 installed in an incubator 5, and is provided with an optical-data acquisition unit 1, a medium changing unit 2, a controlling means 3, the cell-culturing vessel 4, and the incubator 5.

As shown in Fig. 2A, the optical-data acquisition unit 1 is provided with: an irradiation optical system 1a that radiates monochromatic light onto the medium A in the cell-culturing vessel 4; and a measurement optical system 1b that measures a light intensity of the monochromatic light radiated from the irradiation optical system 1a.

The irradiation optical system 1a is provided with a light source 11 that emits the monochromatic light, and the light radiated from the light source 11 is converted to substantially parallel light by a collimating lens 12 and is radiated onto the medium A in the cell-culturing vessel 4.

The measurement optical system 1b is provided with a focusing lens 13 that focuses the monochromatic light radiated from the irradiation optical system 1a and a light-level detector 14 that measures the intensity of the light focused by the focusing lens 13.

The irradiation optical system 1a and the measurement optical system 1b face each other in the top-to-bottom direction with the cell-culturing vessel 4 interposed therebetween, and the monochromatic light emitted from the irradiation optical system 1a is detected by the measurement optical system 1b after passing through the cell-culturing vessel 4. The measurement optical system 1b is accommodated inside a base 17 on which the cell-culturing vessel 4 is mounted. A mounting surface 17a of the base 17 on which the cell-culturing vessel 4 is mounted is formed of an optically transparent member in at least a portion thereof through which the monochromatic light coming from the irradiation optical system 1a passes.

The controlling means 3 is provided with a control portion 15 and a transmitting portion 16. The control portion 15 performs ON/OFF control of the light source 11 and computation of the light intensity measured by the light-level detector 14. The control portion 15 can transmit signals to the medium changing unit 2 via the transmitting portion 16.

The medium changing unit 2 is provided with a medium-supplying means 2a for supplying the medium A to the cell-culturing vessel 4 and a medium-discharging means 2b for discharging the medium A from the cell-culturing vessel 4.

The medium-supplying means 2a is provided with a medium holding means for holding the medium A and a tubular member (a tube or the like) for connecting the medium holding means and the cell-culturing vessel 4, and supplies the medium in the medium holding means to the cell-culturing vessel 4 via the tubular member. The medium holding means is provided with a holding vessel for accommodating the medium A.

A liquid feeding pump (a peristaltic pump or the like) may be installed in the tubular member, and ON and OFF states thereof may be switched between when the liquid feeding pump receives the signals from the transmitting portion 16, thereby controlling the supply of the medium to the cell-culturing vessel 4.

In addition, the medium holding means may be installed above the cell-culturing vessel 4 in the direction of gravity, so that the medium A in the medium holding means may be supplied to the cell-culturing vessel 4 via the tubular member by means of gravity. At this time, a gate for opening/closing the flow channel formed by the tubular member may be installed in the tubular member, and OPEN and CLOSED states of the flow channel may be switched between when the gate receives the signals from the transmitting portion 16, thereby controlling the supply of the medium to the cell-culturing vessel 4. Examples of the gate include a valve.

Fig. 3 shows an example of the medium-supplying means 2a. A holding vessel 21 that can accommodate the medium A in the interior thereof has a discharge port 21a for discharging the medium A. A tubular member 22 connects the discharge port 21a of the holding vessel 21 and a supply port 4a of the cell-culturing vessel 4. A liquid feeding pump 23 is installed in the tubular member 22, and it is possible to supply the medium A in the holding vessel 21 to the cell-culturing vessel 4 via the tubular member 22 by activating the liquid feeding pump 23. Here, the liquid feeding pump 23 has a receiving portion, and ON/OFF control thereof is performed by receiving the signals from the transmitting portion 16.

The medium-discharging means 2b is provided with a waste-liquid holding vessel that holds the medium A discharged from the cell-culturing vessel 4 and a tubular member (tube or the like) that connects the waste-liquid holding vessel and the cell-culturing vessel 4; and discharges the medium A in the cell-culturing vessel 4 into the waste-liquid holding vessel via the tubular member.

The cell-culturing vessel 4 may have an opening 4b on a side surface at a predetermined height from a bottom surface, the tubular member may be connected to the opening 4b, and the medium A may be discharged into the waste-liquid holding vessel from the opening 4b via the tubular member when the medium A in the cell-culturing vessel 4 reaches the opening 4b. Alternatively, the tubular member may be made to protrude into the interior of the cell-culturing vessel 4 from the bottom surface thereof so as to reach a predetermined height and may have an opening at the predetermined height, and the medium A may be discharged into the waste-liquid holding vessel from the opening via the tubular member when the medium A in the cell-culturing vessel 4 reaches the opening of the protruded tubular member.

A liquid feeding pump (a peristaltic pump or the like) may be installed in the tubular member, and ON and OFF states thereof may be switched between when the liquid feeding pump receives the signals from the transmitting portion 16, thereby controlling the medium discharge from the cell-culturing vessel 4.

As shown in Fig. 4, the medium-discharging means 2b may be provided with a negative-pressure supplying means 25 that applies a negative pressure to a waste-liquid holding vessel 24, and ON/OFF control thereof may be performed by a receiving portion of the negative-pressure supplying means 25 by receiving the signals from the transmitting portion 16. By doing so, the negative pressure is transmitted to the cell-culturing vessel 4 via a tubular member 26, and thus, it is possible to suck the medium A into the waste-liquid holding vessel 24 from the cell-culturing vessel 4 by means of the negative pressure, and thus, it is possible to control the discharging of the medium from the cell-culturing vessel 4. The negative-pressure supplying means 25 may be, for example, a pump that sucks out a gas in the waste-liquid holding vessel 24.

The control portion 15 is provided with, for example, a timer (not shown), periodically activates the light source 11 and the light-level detector 14, and calculates an amount of light absorbed by the medium A (absorbance) over time. When the amount of light absorbed by the medium A reaches a threshold that is set in advance, the control portion 15 transmits the signals via the transmitting portion 16. The signals are transmitted to the medium-supplying means 2a and/or the medium-discharging means 2b, and the medium-supplying means 2a and/or the medium-discharging means 2b that have received the signals start medium supply/discharge by using the signals as triggers. The medium-supplying means 2a and/or the medium-discharging means 2b may stop after a certain amount of time has passed, or the control portion 15 may cause the medium-supplying means 2a and/or the medium-discharging means 2b to stop by transmitting the signals via the transmitting portion 16 after a certain amount of time has passed.

The control portion 15 may store the light intensity of the monochromatic light emitted from the light source 11 in advance, and may compute the amount of light absorbed by the medium A on the basis of the stored light intensity and the light intensity measured by the light-level detector 14.

The control portion 15 may determine the timing at which the signals are transmitted from the transmitting portion 16 on the basis of the light intensity of the monochromatic light that has passed through the medium A instead of calculating the amount of light absorbed by the medium A.

### (Second Embodiment)

A cell-culturing apparatus according to a second embodiment of the present invention is an apparatus provided with an optical-data acquisition unit shown in Fig. 5A, and differs from that of the first embodiment in that a measurement optical system 1c is provided on the optical axis that connects the irradiation optical system 1a and the measurement optical system 1b of the cell-culturing apparatus of the first embodiment. The rest of the configuration is similar to that of the first embodiment.

An optical-data acquisition unit 1 of this embodiment is provided with: the irradiation optical system 1a that radiates the monochromatic light onto the medium A in the cell-culturing vessel 4; the measurement optical system (first measurement optical system) 1c that measures the light intensity of a portion of the monochromatic light radiated from the irradiation optical system 1a; and the measurement optical system (second measurement optical system) 1b that measures the light intensity of the other portion of the monochromatic light radiated from the irradiation optical system 1a.

The irradiation optical system 1a is provided with the light source 11 that emits the monochromatic light, and the light radiated from the light source 11 is converted to substantially parallel light by the collimating lens 12 and is radiated toward the medium A in the cell-culturing vessel 4.

The measurement optical system 1c is provided with a half mirror 31, a focusing lens 33, and a light-level detector 34. The half mirror 31 is disposed on the optical axis that connects the irradiation optical system 1a and the measurement optical system 1b, reflects one half of the monochromatic light coming from the irradiation optical system 1a, and allows the remaining half to pass therethrough. Here, the half mirror 31 is disposed on the optical axis before the monochromatic light coming from the irradiation optical system 1a reaches the cell-culturing vessel 4, reflects one half of the monochromatic light toward the light-level detector 34, and allows the remaining half of the monochromatic light to pass therethrough toward the cell-culturing vessel 4. The monochromatic light reflected by the half mirror 31 is focused by the focusing lens 33, and the light intensity thereof is measured by the light-level detector 34.

The measurement optical system 1b is provided with the focusing lens 13 that focuses the monochromatic light radiated from the irradiation optical system 1a and that has passed through the half mirror 31 and the light-level detector 14 that measures the intensity of the light focused by the focusing lens 13; and is accommodated inside the base 17 on which the cell-culturing vessel 4 is mounted. The mounting surface 17a of the base 17 on which the cell-culturing vessel 4 is mounted is formed of an optically transparent member in at least the portion thereof through which the monochromatic light coming from the irradiation optical system 1a passes.

The irradiation optical system 1a and the measurement optical system 1b face each other in the top-to-bottom direction with the cell-culturing vessel 4 interposed therebetween, and the monochromatic light that is emitted from the irradiation optical system 1a and that has passed through the half mirror 31 passes through the cell-culturing vessel 4 and is detected by the light-level detector 14 of the measurement optical system 1b. On the other hand, the monochromatic light that is emitted from the irradiation optical system 1a and that has been reflected by the half mirror 31 is detected by the light-level detector 34 of the measurement optical system 1c without passing through the cell-culturing vessel 4.

The controlling means 3 is provided with the control portion 15 and the transmitting portion 16. The control portion 15 performs ON/OFF control of the light source 11 and computation of the light intensity measured by the light-level detector 14 and the light-level detector 34. The control portion 15 can transmit the signals to the medium changing unit 2 via the transmitting portion 16.

The control portion 15 can calculate the amount of light absorbed by the medium A by using the light intensities measured by the measurement optical system 1b and the measurement optical system 1c. In other words, the amount of monochromatic light absorbed by the medium A is calculated on the basis of a difference between the light intensity measured by the measurement optical system 1c and the light intensity measured by the measurement optical system 1b.

The control of the medium changing unit 2 performed by the control portion 15 is similar to that in the first embodiment.

In this embodiment, although the form in which the measurement optical system 1c is provided with the half mirror 31 has been described, so long as a beam splitter that splits light into a reflection direction and a transmission direction by a certain proportion is provided, this component does not need to be the half mirror 31. In this case, the control portion 15 may perform computation by taking the splitting proportion of the beam splitter into consideration, and thus, the amount of light absorbed by the medium A may be calculated. In other words, it suffices that the beam splitter be a means for extracting a portion of the incident light, and this component may be a means for spatially splitting the incident light such as a mirror that reflects half of the beam diameter of the incident light or the like.

### (Third Embodiment)

As shown in Fig. 6A, a cell-culturing apparatus according to a third embodiment of the present invention differs from that of the first embodiment in that a driving means 41 is provided, which moves, relative to the cell-culturing vessel 4, the irradiation optical system 1a and the measurement optical system 1b of the cell-culturing apparatus of the first embodiment. The rest of the configuration is similar to that of the first embodiment.

The driving means 41 is controlled by the control portion 15, and can move the irradiation optical system 1a (the light source 11 and the collimating lens 12) and the measurement optical system 1b (the focusing lens 13 and the light-level detector 14) as a single unit in a horizontal direction (a direction that is orthogonal to the optical axis that connects the irradiation optical system 1a and the measurement optical system 1b).

The driving means 41 may be provided with, for example, a linear motion mechanism that includes a ball screw and may move the irradiation optical system 1a and the measurement optical system 1b along a guide rail or the like by converting a rotational motion to a linear motion by rotating the ball screw by means of a motor or the like.

In addition, the driving means 41 may be provided with, for example, a pulley and a belt (a wire, a chain) and may move the irradiation optical system 1a and the measurement optical system 1b along a guide rail or the like by converting a rotational motion to a linear motion via the belt (a wire, a chain) by applying a rotational force to the pulley by means of a motor or the like.

The controlling means 3 is provided with the control portion 15 and the transmitting portion 16. By moving the irradiation optical system 1a and the measurement optical system 1b by means of the driving means 41, the control portion 15 acquires the light intensity (first light intensity) of the monochromatic light measured, without passing through the cell-culturing vessel 4, at a location at which the optical axis is moved off from the cell-culturing vessel 4, and the light intensity (second light intensity) of the monochromatic light measured, via the cell-culturing vessel 4, at a location at which the cell-culturing vessel 4 is placed on the optical axis. The control portion 15 can calculate the amount of light absorbed by the medium A by using the two light intensities. In other words, it is possible to calculate the amount of monochromatic light absorbed by the medium A on the basis of a difference between the first light intensity and the second light intensity.

The control of the medium changing unit 2 performed by the control portion 15 is similar to that in the first embodiment.

In this embodiment, although the form in which the irradiation optical system 1a and the measurement optical system 1b are moved by means of the driving means 41 has been described, a driving means for moving the cell-culturing vessel 4 may be provided. In that case, a stage on which the cell-culturing vessel 4 is mounted may be provided, and the stage may be moved by means of the driving means.

In the above-described individual embodiments and modifications thereof, although the form in which the irradiation optical system 1a is provided with the light source 11 that emits the monochromatic light and the collimating lens 12 has been described, for example, as shown in Fig. 7A, it is permissible to employ a form in which a band-pass filter 52 that allows light having a specific wavelength to pass therethrough is disposed after a white light source 51 and the collimating lens 12. In that case, the band-pass filter 52 may be changeable, and a band-pass filter that allows light having a desired wavelength to pass therethrough may be insertable into/removable from the optical path of white light coming from the white light source 51.

In addition, it is permissible to employ a form in which the irradiation optical system 1a is provided with a plurality of monochromatic light sources, and a desired monochromatic light source is turned on by switching among the light sources. For example, as shown in Fig. 7B, the irradiation optical system 1a may be provided with three monochromatic light sources 53a, 53b, and 53c that emit light having different wavelengths; optical paths of the light coming from the individual light sources 53a, 53b, and 53c may be combined by providing a mirror 54 and dichroic mirrors 55a and 55b; and monochromatic light having a desired wavelength may be radiated by turning on a desired monochromatic light source. In this case, the control portion 15 may perform computation on the basis of the light-absorption amounts at the multiple wavelengths (for example, calculating ratios of light-absorption amounts at multiple wavelengths or the like), and may determine the timing at which the signals are to be transmitted via the transmitting portion 16.

In the above-described individual embodiments and modifications thereof, examples of the light source that emits the monochromatic light include an LED, an LD, and so forth, and it is possible to use a light source that emits light having a predetermined relatively narrow wavelength width. In addition, light having a desired wavelength may be extracted and radiated by causing the light radiated from the white light source to pass through a narrow band-pass filter. It suffices that the light source emit light having a wavelength width that allows measurement of an absorbance thereof.

In the above-described individual embodiments and modifications thereof, examples of the light-level detector include a photodiode (PD), a photomultiplier tube (PMT), and so forth.

In the above-described individual embodiments and modifications thereof, depending on the light source to be used, there are cases in which the collimating lens 12 of the irradiation optical system 1a is not necessary. In addition, depending on the light-level detector to be used, there are cases in which the focusing lens 13 of the measurement optical system 1b is not necessary.

In the above-described individual embodiments and modifications thereof, although the form in which the controlling means 3 transmits the signals to the medium changing unit 2 has been described, for example, as shown in Fig. 8A, the transmitting portion 16 of the controlling means 3 may serve as a transmitting/receiving portion 16a that can receive external signals. The transmitting/receiving portion 16a may transmit/receive signals to/from an external controlling means 60 installed outside the incubator 5, and the measurement of the absorbance and the medium change may be remotely controlled. In this case, it is permissible that the control portion 15 is not provided with a timer.

In addition, for example, as shown in Fig. 8B, the external controlling means 60 may directly control the optical-data acquisition unit 1 and the medium changing unit 2 without providing the controlling means 3.

Examples of the external controlling means 60 include a personal computer (PC). For example, a PC may include a CPU and a memory, and may realize an external controlling means function by executing a control program stored in the memory by means of the CPU. The measurement of the absorbance and the medium change may be remotely controlled by an operator operating the PC.

In the above-described individual embodiments and modifications thereof, the optical-data acquisition unit 1 and the cell-culturing vessel 4 are disposed inside the incubator 5. The medium changing unit 2 may be disposed inside the incubator 5, or a portion thereof may be disposed outside the incubator 5. The controlling means 3 may be disposed inside the incubator 5 or may be disposed outside the incubator 5.

In the above-described individual embodiments and modifica tions thereof, although the form in which the monochromatic ligh t is radiated by the irradiation optical system 1a toward the bo ttom surface of the cell-culturing vessel 4 from a top surface t hereof has been described, it is permissible to employ a form in which the irradiation optical system 1a and the measurement opt ical system 1b are disposed at vertically inverted positions wit h the cell-culturing vessel 4 interposed therebetween and the mo nochromatic light is radiated toward the top surface of the cell -culturing vessel 4 from the bottom surface thereof.

In addition, for example, as shown in Fig. 2B, the irradiation optical system 1a and the measurement optical system 1b may be disposed in a horizontal direction with the cell-culturing vessel 4 interposed therebetween, and the monochromatic light may be radiated onto the medium A from a side surface of the cell-culturing vessel 4. By doing so, it is possible to measure the amount of light absorbed by the medium A at a position in the cell-culturing vessel 4 at which the cells are absent, and thus, it is possible to eliminate the light absorption by the cells. Figs. 2B, 5B, and 6B show modifications that correspond to the first embodiment, the second embodiment, and the third embodiment, respectively.

In the above-described individual embodiments and modifica tions thereof, although the form in which the irradiation optica l system 1a and the measurement optical system 1b are disposed w ith the cell-culturing vessel 4 interposed therebetween has bee n described, for example, as shown in Fig. 10A, the irradiation optical system 1a (a light source 71 and a collimating lens 72) and the measurement optical system 1b (a focusing lens 73, a lig ht-level detector 74, and a half mirror 79) may be disposed on t he same side with respect to the cell-culturing vessel 4, and a reflecting member 78 may be disposed on a side at which the refl ecting member 78 faces the irradiation optical system 1a and the measurement optical system 1b, with the cell-culturing vessel ( not shown) interposed therebetween.

In this case, the light radiated from the light source 71 that emits the monochromatic light is converted to substantially parallel light by means of the collimating lens 72, and half of the monochromatic light that has passed through the half mirror 79 is radiated onto the medium A in the cell-culturing vessel. The monochromatic light that has passed through the cell-culturing vessel is reflected by the reflecting member 78 disposed above the cell-culturing vessel and passes through the cell-culturing vessel again. With the monochromatic light that has passed through the cell-culturing vessel, half thereof is reflected by the half mirror 79, this light is focused by the focusing lens 73, and the intensity thereof is measured by the light-level detector 74.

The irradiation optical system (the light source 71 and the collimating lens 72) and the measurement optical system (the fo cusing lens 73, the light-level detector 74, and the half mirror 79) are accommodated inside a base 77 on which the cell-culturi ng vessel is mounted. A mounting surface 77a of the base 77 on which the cell-culturing vessel is mounted is formed of an optic ally transparent member in at least a portion thereof through wh ich the monochromatic light passes. The controlling means (a co ntrol portion 75 and a transmitting portion 76) may also be acco mmodated inside the base 77.

Although Fig. 10A shows the form in which the reflecting member 78 is integrally attached to the base 77, the reflecting member 78 may be provided as a separate component. A cell-culturing vessel in which the reflecting member 78 is attached to a top surface thereof may be used. The reflecting member 78 is, for example, a mirror.

In addition, as shown in Fig. 10B, it is permissible to employ a form in which the irradiation optical system (the light source 71 and the collimating lens 72) and the measurement optical system (the focusing lens 73, the light-level detector 74, and the half mirror 79) are disposed at a side surface of the cell-culturing vessel (not shown).

By employing such a form, it is possible to make the apparatus configuration compact, and it is made easier to dispose the apparatus in the incubator. In addition, because the monochromatic light is made to pass through the medium A in the cell-culturing vessel twice, the amount of light absorbed by the medium A is increased, and thus, the sensitivity for detecting changes in the light-absorption amount is increased.

Instead of the half mirror 79, a beam splitter that splits light into a reflection direction and a transmission direction by a certain proportion may be employed. In this case, the control portion 75 may perform computation by taking the splitting proportion of the beam splitter into consideration, and thus, the amount of light absorbed by the medium A may be calculated. In other words, a means for extracting a portion of the incident light may be employed instead of the half mirror 79, and a means for spatially splitting the incident light, such as a mirror that reflects a half of a beam diameter of the incident light or the like, may be employed.

Figs. 11A and 11B show modifications of the forms in Figs. 10A and 10B. These modifications are additionally provided with a half mirror 80 that is disposed so as to be switchable with t he half mirror 79. The half mirror 79 and the half mirror 80 ar e disposed so as to be inclined at mutually orthogonal angles, a nd can be switched on the optical axis by means of a driving mec hanism (not shown).

With these forms, it is possible to measure the light level of the monochromatic light that has passed through the cell-culturing vessel in a state in which the half mirror 79 is disposed in the optical path, and it is possible to measure the light level of the monochromatic light that has not passed through the cell-culturing vessel in a state in which the half mirror 80 is disposed in the optical path. The control portion 75 can calculate the amount of light absorbed by the medium A on the basis of the two types of the acquired light-level data and the proportions by which the monochromatic light is split by the half mirrors 79 and 80.

Instead of switching the half mirror 79 and the half mirror 80, the angle at which the half mirror 79 is disposed may be rotated by 90° by means of a driving mechanism (not shown).

In this modification also, instead of the half mirror 79, a beam splitter that splits light into a reflection direction and a transmission direction by a certain proportion may be employed. In this case, the control portion 75 may perform computation by taking the splitting proportion of the beam splitter into consideration, and thus, the amount of light absorbed by the medium A may be calculated. In other words, a means for extracting a portion of the incident light may be employed instead of the half mirror 79, and a means for spatially splitting the incident light, such as a mirror that reflects a half of a beam diameter of the incident light or the like, may be employed.

Although Figs. 10A, 10B, 11A, and 11B show the forms in which the monochromatic light radiated from the light source 71 passes through the medium A in the cell-culturing vessel 4 twice via the reflecting member 78, a form in which the monochromatic light passes through the medium A in the cell-culturing vessel 4 three times or more may be employed.

For example, as shown in Fig. 12A, by making the monochroma tic light radiated from the light source 71 incident on a reflec ting surface of a reflecting member 78A at an angle, the reflect ed monochromatic light is made to reach the base 77 at a positio n that is displaced from the center (optical axis) of the light source 71. In order to make the monochromatic light incident on the reflecting surface, the optical axis of the light source 71 may be inclined, and the reflecting surface of the reflecting m ember 78A may be inclined. Another reflecting member 78B is dis posed at a position at which the monochromatic light reaches, an d the monochromatic light is reflected by the reflecting member 78B, thus passing through the medium A in the cell-culturing ves sel 4 again. The focusing lens 73 and the light-level detector 74 are disposed in the optical path of the monochromatic light t hat has passed through the medium A again from the light source 71 side, and thus, the light level of the monochromatic light th at has passed through the medium A in the cell-culturing vessel 4 three times is measured by means of the focusing lens 73 and t he light-level detector 74.

Furthermore, as shown in Fig. 12B, after the monochromatic light is reflected downward by a reflecting member 78C (the reflecting member 78A can be used so as to serve also as the reflecting member 78C) that is disposed above the cell-culturing vessel 4 and the light is made to pass through the medium A in the cell-culturing vessel 4 again, by measuring the light level of the monochromatic light by means of the focusing lens 73 and the light-level detector 74 that are disposed in the optical path of the monochromatic light that has passed through the medium A downward, it is possible to measure the light level of the light that has passed through the medium A in the cell-culturing vessel 4 four times.

As has been described above, by disposing the reflecting members 78A, 78C and the reflecting member 78B above and below the cell-culturing vessel 4, respectively, and by measuring the light level by means of the focusing lens 73 and the light-level detector 74 disposed in the optical path after the monochromatic light is made to pass through the medium A in the cell-culturing vessel 4 multiple times by reflecting the monochromatic light between the respective reflecting members multiple times, it is possible to measure the light level of the light that has passed through the medium A in the cell-culturing vessel 4 multiple times. In a form in which the monochromatic light is reflected an odd number of times, the focusing lens 73 and the light-level detector 74 are disposed above the cell-culturing vessel 4, and, in a form in which the monochromatic light is reflected an even number of times, the focusing lens 73 and the light-level detector 74 are disposed below the cell-culturing vessel 4.

With such forms, because the distance by which the monochromatic light passes through the medium A in the cell-culturing vessel 4 is increased, the amount of light absorbed by the medium A is further increased, and thus, it is possible to further enhance the detection sensitivity for changes in the light-absorption amount.

In the above-described individual embodiments and modifications thereof, the transmission by the transmitting portion 16 may be performed wiredly or wirelessly. In addition, the transmission/reception of the signals between the external controlling means 60 and the transmitting portion 16 or the transmitting/receiving portion 16a may be performed wiredly or wirelessly.

Examples of the cell-culturing vessel 4 include a flask, a petri dish, a culturing bag, and a reactor (a culturing tank).

With the present invention, it is possible to provide a cell-culturing system 200 including: a cell-culturing apparatus (first cell-culturing apparatus) 61 according to any one of the above-described individual embodiments and modifications thereof; and a cell-culturing apparatus (second cell-culturing apparatus) 62 that does not include the optical-data acquisition unit 1 and the controlling means 3 and that includes the medium changing unit (second medium changing unit) 2 and the cell-culturing vessel (second cell-culturing vessel) 4.

In this cell-culturing system 200, the optical-data acquisition unit 1 of the first cell-culturing apparatus 61 measures the amount of light absorbed by the medium A over time, and the controlling means 3 transmits the signals to the medium changing units 2 of the first cell-culturing apparatus 61 and the second cell-culturing apparatus 62 when the amount of light absorbed by the medium A reaches a threshold set in advance. The individual medium changing units 2 that have received the signals start medium supply/discharge by using the signals as triggers.

For example, as shown in Fig. 9, multiple units of the second cell-culturing apparatus 62 may be provided. In this case, the controlling means 3 of the first cell-culturing apparatus 61 can transmit the signals to the medium changing units 2 of the first cell-culturing apparatus 61 and the individual second cell-culturing apparatuses 62, and the individual medium changing units 2 that have received the signals start medium supply/discharge by using the signals as triggers.

In the present invention, it is preferable that the light absorption by phenol red added to the medium A be measured. Because phenol red has absorption peaks in the vicinity of 430 nm and the vicinity of 560 nm, it is preferable that monochromatic light having wavelengths in the vicinities thereof be used.

With the present invention, it is possible to provide
a light-absorption measuring apparatus provided with: an optical-data acquisition unit that acquires optical data of a medium in a cell-culturing vessel; and a controlling means,
wherein the optical-data acquisition unit is provided with an irradiation optical system that radiates monochromatic light onto the medium in the cell-culturing vessel, a beam splitter that splits the monochromatic light radiated from the irradiation optical system into two beams, a first measurement optical system that, of the monochromatic light beams split by the beam splitter, measures a light intensity of one of the monochromatic light beams before passing through the medium in the cell-culturing vessel, and a second measurement optical system that, of the monochromatic light beams split by the beam splitter, measures a light intensity of the other monochromatic light beam that has passed through the medium in the cell-culturing vessel, and
wherein the controlling means measures an amount of light absorbed by the medium on the basis of the light intensities measured by the first measurement optical system and the second measurement optical system.

With the present invention, it is possible to provide a light-absorption measuring apparatus including:
an optical-data acquisition unit that acquires optical data of a medium in a cell-culturing vessel;
a driving means for moving the optical-data acquisition unit and the cell-culturing vessel relative to each other; and a controlling means,
wherein the optical-data acquisition unit is provided with an irradiation optical system that radiates monochromatic light onto the medium in the cell-culturing vessel, and a measurement optical system that measures the light intensity of the monochromatic light radiated from the irradiation optical system, and
wherein the controlling means causes the optical-data acquisition unit and the cell-culturing vessel to be moved relative to each other by means of the driving means, and measures the amount of light absorbed by the medium on the basis of a first light intensity measured when the cell-culturing vessel is not disposed in an optical path between the irradiation optical system and the measurement optical system and a second light intensity measured when the cell-culturing vessel is disposed in the optical path between the irradiation optical system and the measurement optical system.

With the present invention, it is possible to provide a cell-culturing vessel that holds cells and a medium in an internal space thereof, the cell-culturing vessel including: a supply port for supplying the medium into the internal space; a discharge port that is provided at a bottom portion of the vessel and from which the medium is discharged from the internal space; and a discharge mechanism that forms a flow channel that protrudes upward into the internal space from the discharge port so as to reach a predetermined height and to provide an opening thereat.

With this cell-culturing vessel, when the height of the medium accumulated in the internal space is increased by the medium being supplied from the supply port and when the height thereof reaches the opening of the discharge mechanism, the amount of medium exceeding that height is discharged from the internal space via the opening. It is preferable that the positions of the supply port and the opening of the discharge mechanism be separated from each other because doing so increases the medium-changing efficiency.

### {Reference Signs List}

- 1: optical-data acquisition unit
- 1a: irradiation optical system
- 1b: measurement optical system
- 2: medium changing unit
- 3: controlling means
- 4: cell-culturing vessel
- 5: incubator
- 11, 71: light source
- 12, 72: collimating lens
- 13, 33, 73: focusing lens
- 14, 34, 74: light-level detector
- 15, 75: control portion
- 16, 76: transmitting portion
- 17, 77: base
- 21: holding vessel
- 22, 26: tubular member
- 23: liquid feeding pump
- 24: waste-liquid holding vessel
- 25: negative-pressure supplying means
- 31, 79, 80: half mirror
- 41: driving means
- 51: white light source
- 52: band-pass filter
- 53a, 53b, 53c: monochromatic light source
- 54: mirror
- 55a, 55b: dichroic mirror
- 60: external controlling means
- 61: first cell-culturing apparatus
- 62: second cell-culturing apparatus
- 78: reflecting member

## Claims

1. A cell-culturing apparatus comprising:
an incubator that can maintain an interior thereof in an environment that is appropriate for growth of cells;
a cell-culturing vessel that is accommodated inside the incubator and that holds cells and a medium therein;
an optical-data acquisition unit that acquires optical data of the medium in the cell-culturing vessel;
a medium changing unit that changes the medium in the cell-culturing vessel; and
a controlling means,
wherein the optical-data acquisition unit is provided with an irradiation optical system that radiates monochromatic light onto the medium in the cell-culturing vessel and a measurement optical system that measures a light intensity of the monochromatic light that has passed through the medium in the cell-culturing vessel,
wherein the medium changing unit is provided with a medium-supplying means for supplying the medium to the cell-culturing vessel and a medium-discharging means for discharging the medium from the cell-culturing vessel, and
wherein the controlling means detects a change over time in the light intensity measured by the measurement optical system, and controls the medium-supplying means and/or the medium-disc harging means on the basis of said change over time.

2. A cell-culturing apparatus comprising:
an incubator that can maintain an interior thereof in an environment that is appropriate for growth of cells;
a cell-culturing vessel that is accommodated inside the incubator and that holds cells and a medium therein;
an optical-data acquisition unit that acquires optical data of the medium in the cell-culturing vessel;
a medium changing unit that changes the medium in the cell-culturing vessel; and
a controlling means,
wherein the optical-data acquisition unit is provided with an irradiation optical system that radiates monochromatic ligh t onto the medium in the cell-culturing vessel, a beam splitter that splits the monochromatic light radiated from the irradiati on optical system into two beams, a first measurement optical sy stem that, of the monochromatic light beams split by the beam sp litter, measures a light intensity of one of the monochromatic 1 ight beams before passing through the medium in the cell-culturi ng vessel, and a second measurement optical system that, of the monochromatic light beams split by the beam splitter, measures a light intensity of the other monochromatic light beam that has passed through the medium in the cell-culturing vessel,
wherein the medium changing unit is provided with a medium-supplying means for supplying the medium to the cell-culturing vessel and a medium-discharging means for discharging the medium from the cell-culturing vessel, and
wherein the controlling means calculates an amount of ligh t absorbed by the medium on the basis of the light intensities m easured by the first measurement optical system and the second m easurement optical system, detects a change over time in the cal culated light-absorption amount, and controls the medium-supply ing means and/or the medium-discharging means on the basis of sa id change over time.

3. A cell-culturing apparatus comprising:
an incubator that can maintain an interior thereof in an environment that is appropriate for growth of cells;
a cell-culturing vessel that is accommodated inside the incubator and that holds cells and a medium therein;
an optical-data acquisition unit that acquires optical data of the medium in the cell-culturing vessel;
a driving means for moving the optical-data acquisition unit and the cell-culturing vessel relative to each other;
a medium changing unit that changes the medium in the cell-culturing vessel; and
a controlling means,
wherein the optical-data acquisition unit is provided with an irradiation optical system that radiates monochromatic light onto the medium in the cell-culturing vessel and a measurement optical system that measures a light intensity of the monochromatic light radiated from the irradiation optical system,
wherein the medium changing unit is provided with a medium-supplying means for supplying the medium to the cell-culturing vessel and a medium-discharging means for discharging the medium from the cell-culturing vessel, and
wherein the controlling means causes the optical-data acquisition unit and the cell-culturing vessel to be moved relative to each other by means of the driving means, calculates an amount of light absorbed by the medium on the basis of a first light intensity measured when the cell-culturing vessel is not disposed in an optical path between the irradiation optical system and the measurement optical system and a second light intensity measured when the cell-culturing vessel is disposed in the optical path between the irradiation optical system and the measurement optical system, detects a change over time in the calculated light-absorption amount, and controls the medium-supplying means and/or the medium-discharging means on the basis of said change over time.

4. A cell-culturing apparatus according to any one of Claims 1 to 3,
wherein at least the cell-culturing vessel, the optical-data acquisition unit, and the controlling means are disposed inside the incubator, and
wherein the controlling means is provided with a transmitting/receiving portion that transmits/receives signals to/from outside the incubator, and, by transmitting/receiving, by means of the transmitting/receiving portion, the signals to/from an external controlling means installed outside the incubator, the optical-data acquisition unit and the medium changing unit are remotely controlled.

5. A cell-culturing apparatus according to any one of Claims 1 to 4, wherein the irradiation optical system and the measurement optical system are disposed in a top-to-bottom direction with the cell-culturing vessel interposed therebetween.

6. A cell-culturing apparatus according to any one of Claims 1 to 4, wherein the irradiation optical system and the measurement optical system are disposed in a horizontal direction with the cell-culturing vessel interposed therebetween.

7. A cell-culturing apparatus according to any one of Claims 1 to 4,
wherein the irradiation optical system and the measurement optical system are disposed on the same side with respect to the cell-culturing vessel,
wherein a reflecting member is provided on an opposite side of the irradiation optical system and the measurement optical system with the cell-culturing vessel interposed therebetween, and
wherein the measurement optical system measures a light intensity of the monochromatic light that is radiated onto the cell-culturing vessel from the irradiation optical system, that is reflected by the reflecting member after passing through the cell-culturing vessel, and that has passed through the cell-culturing vessel again.

8. A cell-culturing apparatus according to any one of Claims 1 to 7, wherein the irradiation optical system is provided with a white light source, and a band-pass filter that is provided so as to be insertable into/removable from an optical path of white light emitted from the white light source and that allows only light having a desired wavelength to pass therethrough.

9. A cell-culturing apparatus according to any one of Claims 1 to 7, wherein the irradiation optical system is provided with a plurality of monochromatic light sources, and a mirror and a dichroic mirror that combine optical paths of light coming from the plurality of monochromatic light sources.

10. A cell-culturing apparatus according to any one of Claims 1 to 9,
wherein the medium-supplying means is provided with a medium holding means for holding the medium, a tubular member that connects the medium holding means and the cell-culturing vessel, and a liquid feeding pump disposed in the tubular member, and
wherein the liquid feeding pump is controlled by means of the signals from the controlling means.

11. A cell-culturing apparatus according to any one of Claims 1 to 10,
wherein the medium-discharging means is provided with a waste-liquid holding vessel that holds the medium discharged from the cell-culturing vessel, a tubular member that connects the waste-liquid holding vessel and the cell-culturing vessel, and a liquid feeding pump disposed in the tubular member, and
wherein the liquid feeding pump is controlled by means of the signals from the controlling means.

12. A cell-culturing apparatus according to any one of Claims 1 to 10,
wherein the medium-discharging means is provided with a waste-liquid holding vessel that holds the medium discharged from the cell-culturing vessel, a tubular member that connects the waste-liquid holding vessel and the cell-culturing vessel, and a negative-pressure supplying means for applying a negative pressure to the waste-liquid holding vessel, and
wherein the negative-pressure supplying means is controlled by means of the signals from the controlling means.

13. A cell-culturing system comprising:
a first cell-culturing apparatus that is selected from the cell-culturing apparatuses according to any one of Claims 1 to 12, and
a second cell-culturing apparatus that is provided with a second cell-culturing vessel that is accommodated inside the incubator of the first cell-culturing apparatus and that holds cells and a medium, and a second medium changing unit that changes the medium in the second cell-culturing vessel,
wherein the controlling means of the first cell-culturing apparatus controls the medium changing unit of the first cell-culturing apparatus and the second medium changing unit of the second cell-culturing apparatus on the basis of the change over time.
